# EUROPEAN PATENT SPECIFICATION

(11) **EP 1 713 429 B1**
(45) Date of publication and mention of the grant of the patent: **16.06.2010**
(21) Application number: 05712418.2
(22) Date of filing: 03.02.2005
(51) Int. Cl.: A61H 33/06, A61H 33/02

(54) **AIR BATH**
LUFTBAD
BAIGNOIRE A JETS D'AIR

(30) Priority: 06.02.2004 US 774123
(43) Date of publication of application: 25.10.2006
(73) Proprietor: Kohler Co., Kohler WI 53044 (US)
(72) Inventor: SWART, Peter W., Oostburg WI 53070 (US); DAIKUBARA, Mike Yoshiaki, Fox Point, WI 53217 (US); WANG, Diana, Sheboygan, WI 53083 (US)
(74) Representative: Ebner von Eschenbach, Jennifer
(86) International application number: PCT/US2005/002969
(87) International publication number: WO 2005/076853

(56) References cited:
- EP-A- 0 154 935
- AU-B2- 545 813
- GB-A- 2 176 101
- US-A- 3 772 714
- US-A- 3 964 472
- US-A- 4 249 522
- US-A1- 2003 233 704
- US-B1- 6 477 724

## Description

### BACKGROUND OF THE INVENTION

The present invention relates to tubs such as bathtubs in which air is bubbled into the water to create a hydrotherapy action.

Therapeutic water baths and pools are well-known. Spas or whirlpool tubs are common examples in which water streams from jets through the walls of the basin flow into the water beneath the surface, usually directed at large muscle areas of a person's body, for example shoulders, back and thighs. The force from the jets "massage" the bather directly as well as agitate the water to provide therapeutic effects for other parts of the body not directly in the path of the jets. U.S. patent 6,185,757 discloses one such bath with water jets.

Although water jets are common, some tubs use air streams to agitate the water. For example, U.S. patent 5,898,958 discloses one tub that introduces water or air streams into the water. A special control and valve arrangement controls the flow air to a sets of jets in the walls of the tub. Air is passed from an air pump through a manifold and several conduits to the jets. A three-way valve is controlled by the operator to pump air only to dedicated air jets, to only a set of water jets where the water is mixed with air, or to both the water and air jets.

The air pump has two speeds for different levels of agitation. The jets are arranged as arrays with multiple columns and rows at a specific locations in the walls of the tub. For example, the water jets are located at the lower lumbar region and the air jets are located at the bather's feet. Thus, while providing targeted massage, this tub maybe somewhat ineffective for full-body therapy. Moreover, the delivery system is very complex and requires several flexible conduits, one for each jet.

U. S. patent 6,317, 903 uses air jets arranged in spaced apart fashion essentially in a single row extending around the walls of the tub. Air (or ozone) is delivered from a blower to the air jets by an air distribution duct secured to the outer surface of the side walls. Provisions for dividing the air flow to body specific zones are also discussed.

Air distribution in this system is less than optimal because the jets farthest from the blower can effectively become blocked by the pressure head of the water contained in the tub, particularly when the blower speed is decreased. Consequently, less than full body treatment may be occur.

Australian patent AU 545 813 uses air jets arranged in two conduits around the walls of a tub. The conduits may be moulded onto the outer surface of the tub. A plurality of air holes are spaced apart in each conduit.

British patent GB 2 176 101 uses one or more chambers that may be integrally formed with a tub to provide air to a sense of spaced apart holes in the tub. U.S. Patent 3,772,714 uses tubing coiled around the wall of a container, the tubing having our holes.

Air distribution in these systems is less than optimal because the air holes are not separated into zones that can be independently controlled. Consequently, less than optimal treatment of specific body areas may occur.

Thus, a need exists for an improved system for infusing bath water with air.

### SUMMARY OF THE INVENTION

The invention provides an air bathtub. The basin has a bottom and side walls with a plurality of air jets arranged in spaced relation in a plurality of rows extending essentially around the entire perimeter of the side walls, with each row at a different height. An air manifold extends around the side walls of the basin in communication with the air jets.

In preferred forms, there are at least three rows of air jets in the lower half of the side walls.
The rows of air jets are spaced apart vertically no less than 1.27cm (1/2 inch), and preferably 1.59cm (5/8 inch). The air jets themselves are less than 0.64cm (1/4 inch) in diameter, and preferably 0.24cm (3/32 inch) to 0.32cm (1/8 inch) in diameter. There are preferably 40 air jets in each row spaced apart about two inches. Also, the air jets of at least one of the rows is vertically staggered with respect to one or more of the other rows.

According to the invention the air manifold is integral with an outer side of the side walls, being either a separate piece mechanically attached to the basin or being formed as a unitary part of the basin. The air manifold has a plurality of air channels, arranged in series to from a ring around the basin. Thus, each air channel is isolated from the others, either permanently or by a valve, and each air channel is in communication air jets in a particular zone of the basin, for example, head, foot, thoracic or lumbar zones. Each zone can then be supplied with air independently, alone or in combination with other zones, as desired by the bather.

A control interface allows the user to quickly and easily adjust the air to one or more zones by the press of a button. The control can also be used to control the speed of the air pump or blower to adjust the air flow rate through the manifold, and thus the amount of bubbles in the water.

Hence, the tubs of the present invention permit air to be supplied all the way around the tub using a low pressure pump or blower. Further, the tubs are resistant to a tendency for"dead spots"to arise in areas of the bath water where little or no air is flowing.

These and other advantages of the invention will be apparent from the detailed description and drawings.

### BRIEF DESCRIPTION OF THE DRAWINGS

FIG. 1 is a perspective view of an air bath having an air system shown without the air delivery system;

FIG. 2 is a side elevational view thereof with a section of an air manifold shown cut-away;

FIG. 3 is a partial sectional view, taken along line 3-3 of FIG. 2, showing rows of air jets;

FIG. 4 is an enlarged partial section view thereof;

FIG. 5 is a planar view showing a hole pattern of the air jets;

FIG. 6 is a top plan view of the bathtub as shown in FIG. 1;

FIG. 7 is a bottom plan view thereof; and

FIG. 8 illustrates schematically the air delivery system in an embodiment of the air bath in which there are multiple zones that can be independently controlled to provide focused air flow if full body treatment is not desired. Figs. 1-7 show examples not covered by the claims.

### DETAILED DESCRIPTION OF THE PREFERRED EMBODIMENTS

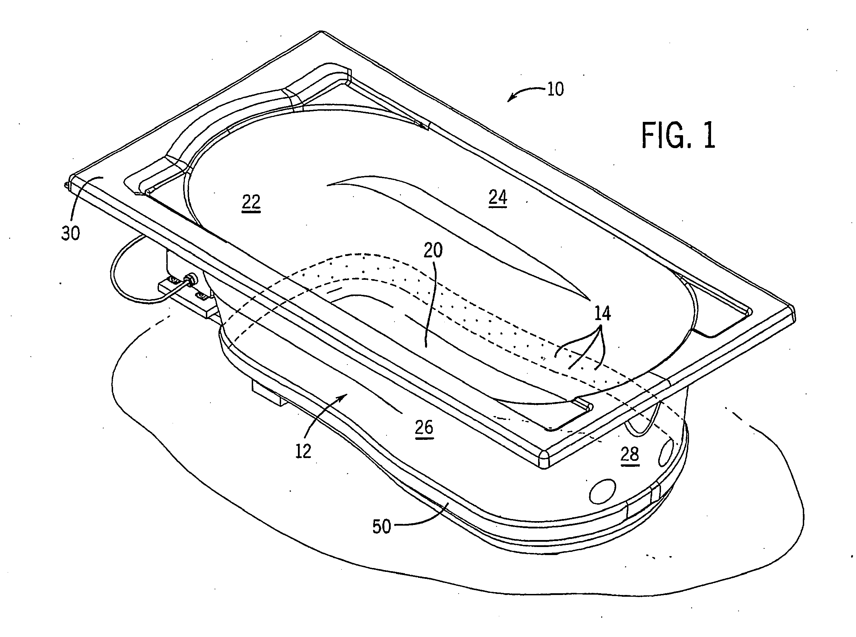

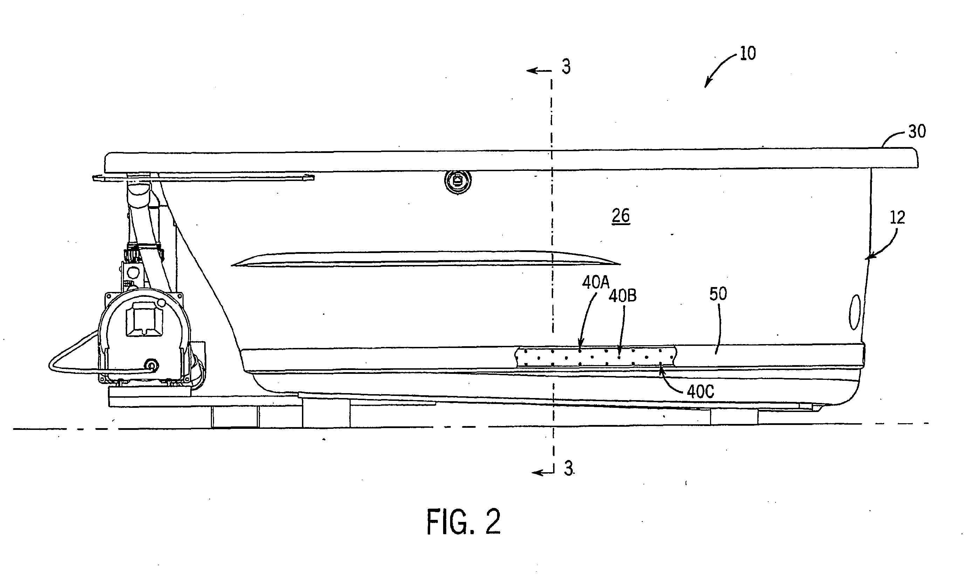

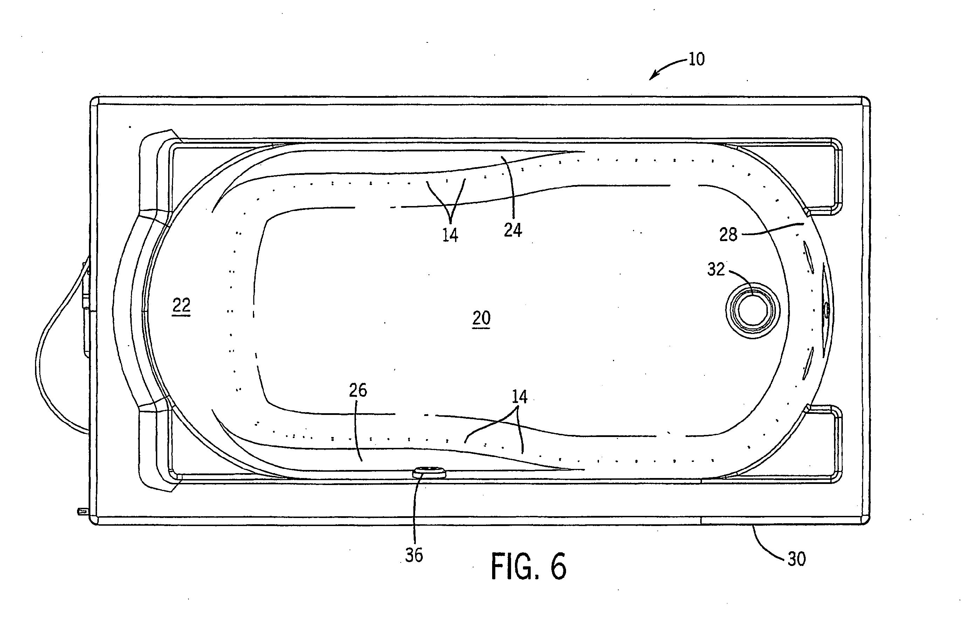

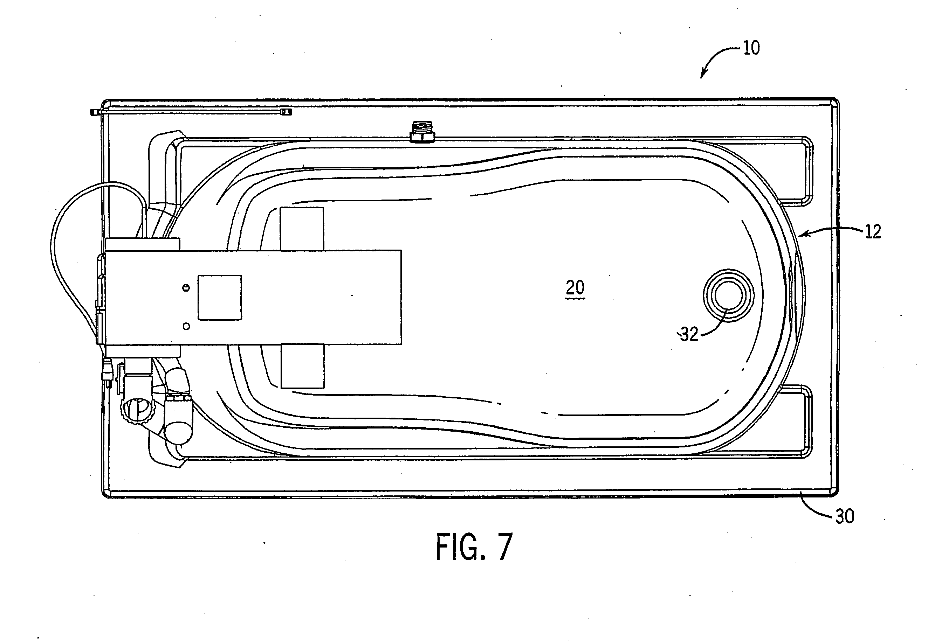

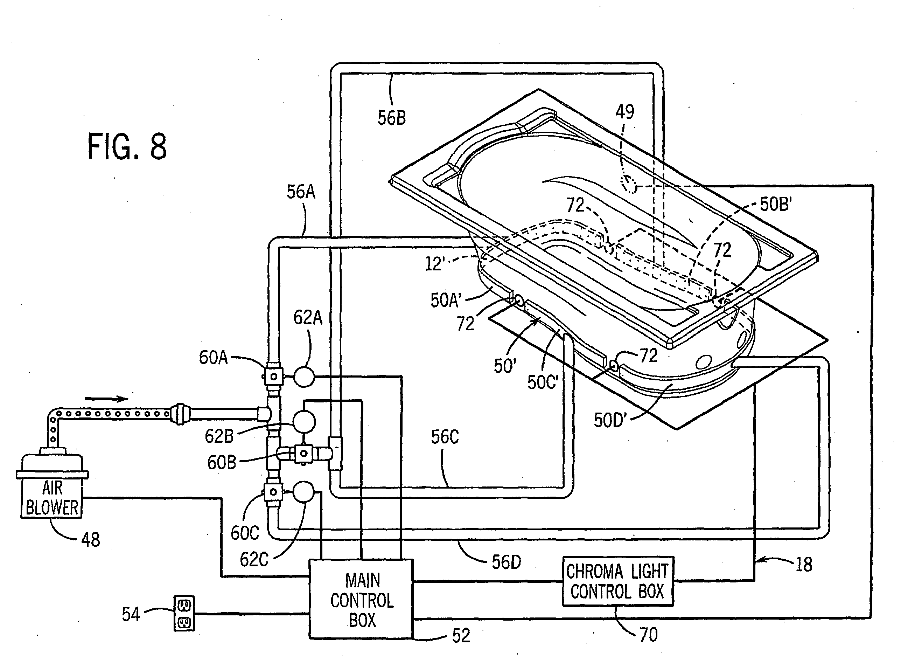

There is disclosed an air bath 10. Figures 1-7 illustrate the configuration of its basin 12 as well as the arrangement of air jets 14. Figure 8 illustrates schematically the air delivery system that can be used with the basin of FIGS. 1-7 as well as the basin 12' shown in FIG. 8. The difference between the two basins is that the basin 12' is specially configured, as will be described, to provide selective control of air flow to prescribed zones, for example, head, lumbar, and foot zones. Figure 8 also illustrates that the air bath can include a chromatherapy system 18 to illuminate the bath water various colors. A suitable chromatherapy system is disclosed in U. S. patent 6,360, 380 and application Ser. No. 10/068,395, filed February 6,2002, both assigned to the assignee of the present invention and hereby incorporated by reference as though fully set t forth herein. Also, in any case, the tub basin can be configured as a conventional bathtub, whirlpool, spa or swimming pool.

Referring to FIGS. 1, 2,6 and 7, the basin 12 of the air bath 10 has a bottom 20 and contoured upright side walls defining a backrest 22, long side walls 24 and 26, and a foot end wall 28. A top ledge 30 extends around the top periphery of the side walls. The bottom 20 has a drain opening 32 with a drain stop controlled remotely by actuator 36 having an overflow feature. The side walls are formed with a plurality of openings near the bottom 20 (or at least somewhere in the lower half of the side walls) that define the air jets 14.

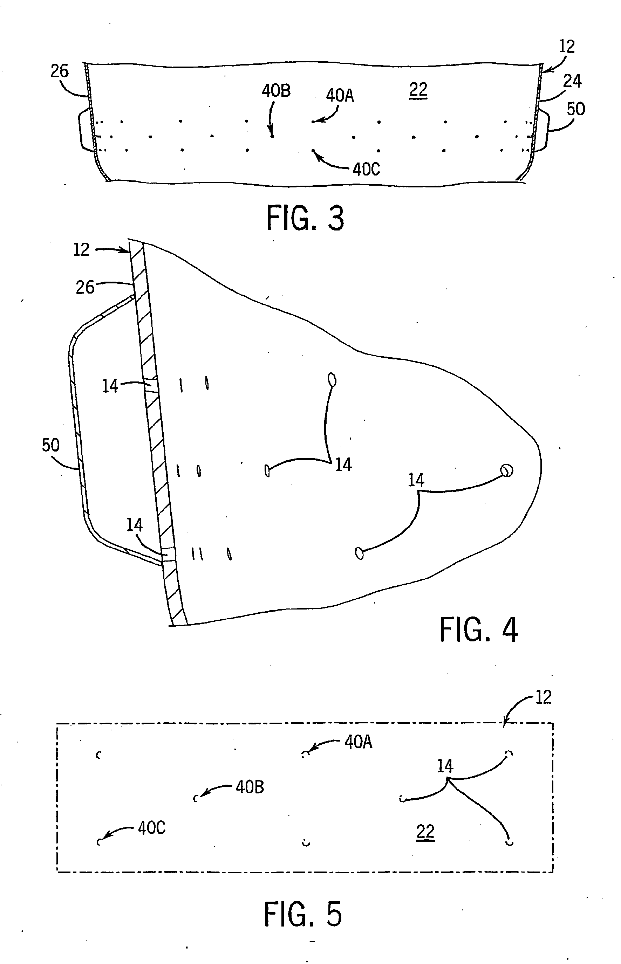

As shown in FIGS. 3-6, the air jets 14 are simple round holes extending though the thickness of the side walls. The air jets themselves are preferably circular and less than 0.64cm (1/4 inch) in diameter, more preferably about 0.24cm (3/32 inch) to 0.32cm (1/8 inch) in diameter. All of the air jets 14 can have an essentially uniform diameter or they can vary by row and/or distance from the air supply.

The openings are arranged in a special pattern designed specifically to provide improved air flow for full body air induced hydrotherapy. The air jets 14 are arranged in a plurality of lateral (or horizontal) rows.
Three rows 40A, 40B and 40C are shown in the drawings.
The air jets 14 of each row are spaced apart from each other, approximately 5.1cm, (two inches), and the air jets 14 of row 40B are staggered vertically so that they are offset from the vertical columns formed by the air jets 14 of rows 40A and 40C (see FIG. 5). In a conventionally sized bathtub, there are preferably about 40 air jets in each row, however, this can vary based on the size of the basin and the spacing between the air jets. Twenty to sixty or more air jets is thus be envisioned. In any case, it is important that the quantity and spacing of the air jets 14 be selected so that the air jets 14 essentially ring the entire perimeter of the side walls so that a full body treatment can be achieved.

All three of the rows 40-40C are near the bottom 20 and preferably do not extend into the upper half of the side walls. More preferably, each row is spaced apart vertically about 1.59cm (5/8 inch). Much less than about 1.27cm (1/2 inch) vertical spacing has been determined to be too small to achieve the desired benefits discussed below. The upper range of the spacing is limited by other factors, such as the height of the side walls, the intended water depth in the basin, whether a single manifold will be used, and if so the ability to achieve sufficient force through the jets without too stringent of air flow requirements.

The vertical spacing of the rows 40A-40C provides an important aspect of the invention. The pressure head created by the water in the basin will be different for air jets at different depths, namely the pressure head being lesser for the higher air jets.
Thus, as is possible in prior art systems, some of the air jets may become effectively blocked by the pressure head, particularly those air jets farthest from the air supply. Unlike prior art systems, however, should this happen in the system of the present invention, air can still flow from air jets in one or more of the higher rows because of the decreased pressure head. Moreover, because the rows of air jets in the present invention extend along the entire perimeter of the side walls, full body treatment can be achieved and maintained without unintended "dead spots" in the water where little or no air flow occurs because the air jets become effectively blocked. This benefit also allows the bather to select more or less agitation without creating dead spots. Dead spots are more likely to occur when the air flow is decreased since less force is generated by the air. So, in conventional systems having adjustable flow control, a bather desiring a more subtle treatment could slow the air flow, however for example, when the desired bubbling at the head was achieved, the water may not be agitated significantly (if at all) at the bather's feet. This problem is avoided by the air jet arrangement of the present invention because in areas where air jets may become blocked by pressure head (such as areas most remote from the air supply) air jets in one or more of the upper rows will still be able to emit air because of the lower pressure head.

With reference to FIGS. 1, 2-4 and 7, air is delivered from an air pump or blower (shown schematically as element 48 in FIG. 8) into an air manifold 50. In the example shown in FIGS. 1-7, the air manifold 50 is a single channel which rings the entire (outer side) perimeter of the side walls such that its interior is in communication with the air jets in all three rows. The embodiment shown in FIG. 8 has an air manifold 50' in which a series of discrete channels are in communication with groups of air jets corresponding to different treatment zones. It is important to note, however, that in either case the single channel or series of channels rings essentially the entire perimeter of the side walls sufficient to achieve full body treatment. Also, in either case, the air manifold can be a separate channel (or channels) mechanically attached and sealed to the basin, or preferably it can be a unitary part of the cast or molded basin. And, in any case, the air manifold has to have at least one fitting for coupling an air line leading from the air blower 48.

The air delivery system will now be discussed in more detail with reference to FIG. 8. As mentioned, FIG. 8 depicts a system with zone control which will be discussed shortly, however, both embodiments include the air blower 48, a user control 49, a controller 52 connected to a power source 54, and air lines 56 coupling the outlet of the air blower 48 to the air manifold 50. The bather can use the control to turn on and off the air blower 48 as well as to adjust the air flow by changing the speed of the air blower 48. In the embodiment shown in FIG. 8, the air delivery system also branches of air lines (56A, 56B, 56C and 56D) for each of the channels (50A', 50B', 50C' and 50D') of the air manifold 50'. Air flow through air line 56A' and channel 50A' is controlled by a butterfly valve 60A. Air flow through both air lines 56B and 56C and respective channels 50B' and 50C' is controlled by a single butterfly valve 60B. Air flow through air line 56D and channel 50D' is controlled by butterfly valve 60C. All of the valves are independently controllable by the controller 52 and electronically actuated actuators 62A-62C connected to the controller 52. The user control for this system can have a touch pad or other button for the bather to select the zone to supply air to, which signals the controller 52 to open or close one or more of the valves 60A-60C. A full-body selection can also be provided on the user control for simultaneously opening all three valves 60A-60C.
Although not shown, one-way check valves can be included in the air delivery system to prevent back flow. And, in the preferred form shown in FIG. 8, the user control and controller 52 can also be used to operate the chromatherapy system 18 (including light box 70 and lights 72 mounted to the side walls) to illuminate the bath water one or more colors.

Preferred embodiments of the invention have been described in detail. However, the invention may be applied in a variety of other embodiments which are within the scope of the invention. Thus, to ascertain the full scope of the invention, the following claims should be referenced.

### INDUSTRIAL APPLICABILITY

The invention provides an air jet system for bathtubs.

## Claims

1. An air bath (10), comprising:
a basin (12) having a bottom (20) and side walls (22,24,26,28);
a plurality of air jets (14) extending through the side walls (22,24,26,28) and arranged in spaced relation in a plurality of rows (40) extending essentially around the perimeter of the side walls (22,24,26,28), wherein each row (40) is at different height; and
an air manifold (50) extending around the side walls (22,24,26,28) of the basin (12) in communication with the air jets (14), wherein the air manifold (50) is integral with an outer side of the side walls (22, 24,26,28) ; **characterised in that**
the air manifold (50) has a plurality of air channels (50A'-50D') arranged in a circuit around the basin (12), wherein each of the air channels (50A'-50D') is in communication with air jets (14) in a zone of the basin (12); and
wherein air flow through each air channel (50A'-50D') is independently controllable.

2. The air bath (10) of claim 1, wherein there are three rows (40) of air jets (14).

3. The air bath (10) of claim 1, wherein the rows (40) of air jets (14) are spaced apart vertically no less than 1.27cm (1/2 inch).

4. The air bath (10) of claim 3, wherein the rows (40) of air jets (14) are spaced apart vertically 1.59cm (5/8 inch).

5. The air bath (10) of claim 1, wherein the air jets (14) are less than 0.64cm (1/4 inch) in diameter.

6. The air bath (10) of claim 5, wherein the air jets (14) are 0.24cm (3/32 inch) to 0.32cm (1/8 inch) in diameter.

7. The air bath (10) of claim 1, wherein there are between 20 and 60 air jets (14) in at least one of the rows (40) of air jets (14).

8. The air bath (10) of claim 7, wherein each row (40) of air jets (14) includes 40 air jets (14).

9. The air bath (10) of claim 1, wherein the plurality of rows (40) of air jets (14) are located vertically within a lower half of the side walls (22,24,26,28) of the basin (12).

10. The air bath (10) of claim 1, wherein the air jets (14) of at least one of the rows (40) of air jets (14) is vertically staggered with respect to at least one other of the rows (40) of air jets (14).

11. The air bath (10) of claim 1, wherein groups of air jets (14) correspond to a plurality of zones in the basin (12) and a plurality of air channels (50A'-50D') of the air manifold (50), and wherein air flow through the air channels (50A'-50D') is independently controllable.

12. The air bath (10) of claim 11, further including an operating control (49) for selecting the zone to deliver air to.

13. The air bath (10) of claim 1, further including an air blower (48) and controller (52) for selectively operating the air blower (48).

14. The air bath (10) of claim 13, further including conduit (56) for routing air from the air blower (48) to the air jets (14).

## Patentansprüche

1. Luftbad (10), das folgendes umfasst:
ein Becken (12) mit einem Boden (20) und Seitenwänden (22, 24, 26, 28);
eine Mehrzahl von Luftdüsen (14), die sich durch die Seitenwände (22, 24, 26, 28) erstrecken und in einer Zwischenabstandsanordnung in einer Mehrzahl von Reihen (50) angeordnet sind, die sich im Wesentlichen um den Perimeter der Seitenwände (22, 24, 26, 28) erstrecken, wobei sich jede Reihe (40) auf einer anderen Höhe befindet; und
einen Luftverteiler (50), der sich um die Seitenwände (22, 24, 26, 28) des Beckens (12) erstreckt, in Übertragungsverbindung mit den Luftdüsen (14), wobei der Luftverteiler (50) integral mit einer Außenseite der Seitenwände (22, 24, 26, 28) ausgebildet ist, **dadurch gekennzeichnet, dass**:
der Luftverteiler (50) eine Mehrzahl von Luftkanälen (50A' - 50D') aufweist, die in einem Kreis um das Becken (12) angeordnet sind, wobei jeder der Luftkanäle (50A' - 50D') in Übertragungsverbindung mit Luftdüsen (14) in einer Zone des Beckens (12) befindet; und
wobei die Luftströmung durch jeden Kanal (50A' - 50D') unabhängig voneinander geregelt werden kann.

2. Luftbad (10) nach Anspruch 1, wobei drei Reihen (40) von Luftdüsen (14) vorgesehen sind.

3. Luftbad (10) nach Anspruch 1, wobei die Reihen (40) der Luftdüsen (14) vertikale Zwischenabstände von mindestens 1,27 cm (0,5 Zoll) aufweisen.

4. Luftbad (10) nach Anspruch 3, wobei die Reihen (40) der Luftdüsen (14) vertikale Zwischenabstände von 1,59 cm (0,625 Zoll) aufweisen.

5. Luftbad (10) nach Anspruch 1, wobei die Luftdüsen (14) einen Durchmesser von weniger als 0,64 cm (0,25 Zoll) aufweisen.

6. Luftbad (10) nach Anspruch 5, wobei die Luftdüsen (14) einen Durchmesser zwischen 0,24 cm (0,09375 Zoll) und 0,32 cm (0,125 Zoll) aufweisen.

7. Luftbad (10) nach Anspruch 1, wobei zwischen 20 und 60 Luftdüsen (14) in mindestens einer der Reihen (40) von Luftdüsen (14) vorgesehen sind.

8. Luftbad (10) nach Anspruch 7, wobei jede Reihe (40) von Luftdüsen (14) 40 Luftdüsen (14) aufweist.

9. Luftbad (10) nach Anspruch 1, wobei die Mehrzahl von Reihen (40) von Luftdüsen (14) vertikal in einer unteren Hälfte der Seitenwände (22, 24, 26, 28) des Beckens (12) angeordnet sind.

10. Luftbad (10) nach Anspruch 1, wobei die Luftdüsen (14) der mindestens einen der Reihen (40) von Luftdüsen (14) vertikal gestapelt angeordnet sind im Verhältnis zu mindestens einer anderen der Reihen (40) der Luftdüsen (14).

11. Luftbad (10) nach Anspruch 1, wobei die Gruppen von Luftdüsen (14) einer Mehrzahl von Zonen in dem Becken (12) und einer Mehrzahl von Luftkanälen (50A' - 50D') des Luftverteilers (50) entsprechen, und wobei die Luftströmung durch die Luftkanäle (50A' - 50D') unabhängig voneinander geregelt werden kann.

12. Luftbad (10) nach Anspruch 11, wobei dieses ferner eine Betriebsregelungseinrichtung (49) aufweist, zur Auswahl der Zone, der Luft zugeführt werden soll.

13. Luftbad (10) nach Anspruch 1, wobei dieses ferner ein Luftgebläse (48) und eine Steuereinheit (52) zum selektiven Betrieb des Luftgebläses (48) aufweist.

14. Luftbad (10) nach Anspruch 13, wobei dieses ferner eine Leitung (56) zum Leiten von Luft von dem Luftgebläse (48) zu den Luftdüsen (14) aufweist.

## Revendications

1. Bain d'air (10), comprenant :
une cuvette (12) ayant une partie inférieure (20) et des parois latérales (22, 24, 26, 28) ;
une pluralité de jets d'air (14) s'étendant à travers les parois latérales (22, 24, 26, 28) et agencée dans une relation espacée dans une pluralité de rangées (40) s'étendant essentiellement autour du périmètre des parois latérales (22, 24, 26, 28), dans lequel chaque rangée (40) est une hauteur différente ; et
un collecteur d'air (50) s'étendant autour des parois latérales (22, 24, 26, 28) de la cuvette (12) en communication avec les jets d'air (14), dans lequel le collecteur d'air (50) fait partie intégrante d'un côté extérieur des parois latérales (22, 24, 26, 28) ; **caractérisé en ce que**
le collecteur d'air (50) a une pluralité de canaux d'air (50A'-50D') agencés dans un circuit autour de la cuvette (12), dans lequel chacun des canaux d'air (50A'-50D') est en communication avec les jets d'air (14) dans une zone de la cuvette (12) ; et
dans lequel le flux d'air à travers chaque canal d'air (50A'-50D') peut être contrôlé indépendamment.

2. Bain d'air (10) selon la revendication 1, dans lequel il y a trois rangées (40) de jets d'air (14).

3. Bain d'air (10) selon la revendication 1, dans lequel les rangées (40) de jets d'air (14) sont espacées verticalement de pas moins de 1,27 cm (1/2 pouce).

4. Bain d'air (10) selon la revendication 3, dans lequel les rangées (40) de jets d'air (14) sont espacées verticalement de 1,59 cm (5/8 pouce).

5. Bain d'air (10) selon la revendication 1, dans lequel les jets d'air (14) mesurent moins de 0,64 cm (1/4 pouce) de diamètre.

6. Bain d'air (10) selon la revendication 5, dans lequel les jets d'air (14) mesurent entre 0,24 cm (3/32 pouce) et 0,32 cm (1/8 pouce) de diamètre.

7. Bain d'air (10) selon la revendication 1, dans lequel il y a entre 20 et 60 jets d'air (14) dans au moins une des rangées (40) de jets d'air (14).

8. Bain d'air (10) selon la revendication 7, dans lequel chaque rangée (40) de jets d'air (14) comprend 40 jets d'air (14).

9. Bain d'air (10) selon la revendication 1, dans lequel la pluralité de rangées (40) de jets d'air (14) est située verticalement dans une moitié inférieure des parois latérales (22, 24, 26, 28) de la cuvette (12).

10. Bain d'air (10) selon la revendication 1, dans lequel les jets d'air (14) d'au moins une des rangées (40) de jets d'air (14) sont verticalement en quinconce par rapport à au moins une autre des rangées (40) des jets d'air (14).

11. Bain d'air (10) selon la revendication 1, dans lequel des groupes de jets d'air (14) correspondent à une pluralité de zones dans la cuvette (12) et une pluralité de canaux d'air (50A'-50D') du collecteur d'air (50), et dans lequel le flux d'air à travers les canaux d'air (50A'-50D') peut être contrôlé indépendamment.

12. Bain d'air (10) selon la revendication 11, comprenant en outre une commande de fonctionnement (49) pour sélectionner à la zone à alimenter en air.

13. Bain d'air (10) selon la revendication 11, comprenant en outre une soufflante d'air (48) et un contrôleur (52) pour faire fonctionner la soufflante d'air (48) de façon sélective.

14. Bain d'air (10) selon la revendication 13, comprenant en outre un conduit (56) pour amener l'air de la soufflante d'air (48) jusqu'aux jets d'air (14).
